# EUROPEAN PATENT APPLICATION

(11) **EP 4 850 603 A1**
(43) Date of publication of application: **09.12.2026**
(21) Application number: 25180077.7
(22) Date of filing: 02.06.2025
(51) Int. Cl.: A61B 5/20

(54) **BODY FLUID BALANCE, SYSTEM COMPRISING THE SAME, AND BODY FLUID MEASUREMENT METHOD**

(71) Applicant: DIURIFLUX MEDICAL SA, 1845 Noville (CH)
(72) Inventor: Goy, Nicolas, 1853 Yvorne (CH); Beuret, Philippe, 1845 Noville (CH); Goy, Jean-Jacques, 1409 Chaneaz (CH)
(74) Representative: P&TS SA (AG, Ltd.)

(57) **Abstract**

The present invention relates to a system, a device and a method adapted for monitoring an output flow and/or amount of liquid from one or more patients with regard to an input flow and/or amount of liquid injected to said one or more patient's body.

## Description

### Technical domain

The present invention concerns a device adapted for measuring a flow and/or a volume of a liquid collected in a tank or a reservoir. In particular, the present device allows for precise flow and/or volume measurement of a body fluid. Typically, a flow and/or a volume of urine can be determined with a high accuracy and reliability. The present invention also concerns a system including such a device. The present invention further concerns a method of determining a flow and/or a volume of a liquid.

### Related art

Several devices are developed so as to determine a flow and/or a quantity of a fluid, in particular a body fluid in medical environments. The document US2022/0233120 for example, describes a body fluid meter comprising two parts removably combined.

Despites the constant efforts made to develop such devices, their usage by medical professionally remains low. The amount of body fluids, typically the urine of a patient is usually determined visually by the nurses or other medical agents. This is time consuming and can be partially inaccurate, in particular due to low frequency of measurement.

### Short disclosure of the invention

An aim of the present invention is the provision of a device adapted for precisely and constantly and/or regularly measuring the flow and/or the volume of a fluid feeding a reservoir. The aim is in particular to measure such a flow and/or volume for a body fluid of a patient, typically the urine, in an accurate and reproducible manner.

Another aim of the present invention is to provide an easy-to-use device adapted for precisely measuring the flow and/or the volume of a fluid feeding a reservoir. An objective is indeed to increase the implementation of such a device so as to improve the quality of measurements within hospitals or related environment. Such an implementation further targets a better support for the medical personal, helping in particular saving time and improving efficiency of the medical care. Another objective of the present invention is to provide a tailored and precise device for the measurement of fluid inflow and outflow in the patient

Another aim of the present invention is to provide a system adapted to accurately determine the flow and/or volume of a fluid feeding a reservoir, in particular a body fluid such as urine, in relation with external parameters. The aim is indeed to evaluate the normality or abnormality of such a flow and/or volume of fluid, taking into consideration the context of the monitored patient.

Another aim of the present invention is to provide a method of measuring the flow and/or volume of a fluid feeding a tank or a reservoir. The aim is here to provide a more accurate method of measurement compared to existing techniques. Additionally, the invention aims to present a measurement method that is easier to implement than the solution currently existing.

Another aim is to provide a device adapted for measuring the flow and/or the volume of a fluid feeding a reservoir, which is robust and resilient to external factors, in particular external loads or pressures.

According to the invention, these aims are attained by the object of the independent claims, and further detailed through the claims depending thereof.

With respect to what is known in the art, the invention offers the advantage of providing a reliable and reproducible measurement for determining the flow and/or volume of a fluid, particularly a bodily fluid such as urine, within a specified time frame. The present invention is particularly suitable for intensive care. Furthermore, the present invention supports medical professionals in their daily work, thanks to its « user friendly » design.

### Short description of the drawings

Exemplar embodiments of the invention are disclosed in the description and illustrated by the following drawings:
- Figure 1 : Representation in front perspective of a device according to an example of the present invention,
- Figure2 : Representation in front perspective of a device, without the reservoir, according to an example of the present invention,
- Figure 3: Representation in back perspective of a device according to an example of the present invention,
- Figure 4: Representation of the details of the flowmeter module according to an embodiment of the present invention,
- Figure 5: Representation of the details of the dynamometer according to an embodiment of the present invention,
- Figure 6 : representation of the details of the intermediate element according to an embodiment of the present invention,
- Figure 7: schematic representation of a system according to an embodiment of the present invention,

### Examples of embodiments of the present invention

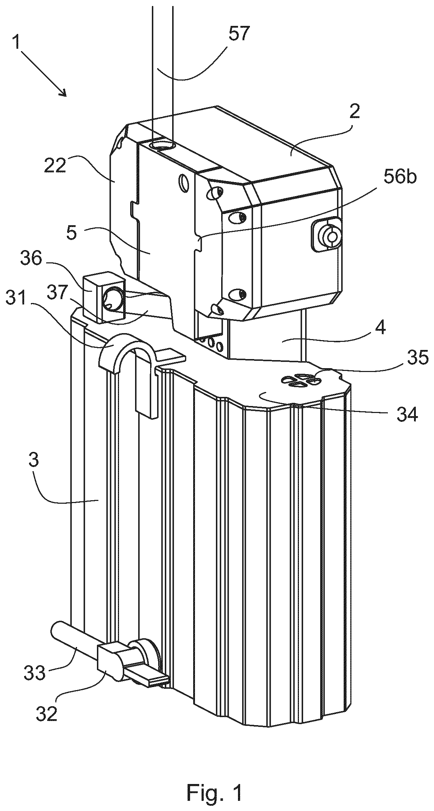

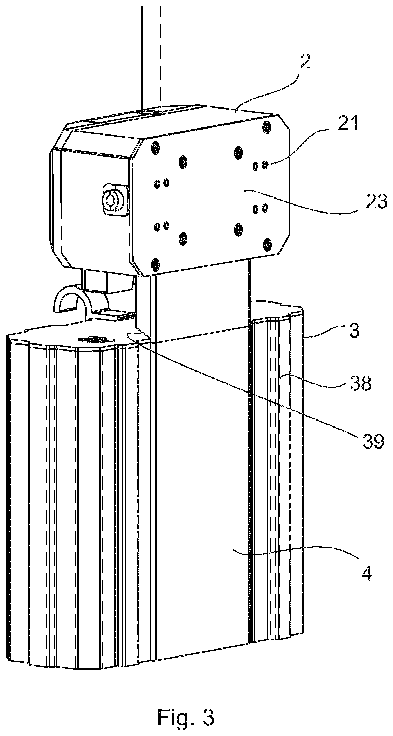

With reference to figure 1, the device **1** according to the present invention comprises an upper element **2,** adapted to be suspended or fixed on an external support. By this means, the upper element **2** can be fixed and immobilized on a wall or on a piece of furniture such as a bed. This is particularly convenient in the hospital environment, wherein the present device can be affixed to the bed of a patient, allowing for example to accompany the patient when the bed is moved from a place to another one. To this end, the upper element **2** comprises one or several upper fixation means **21** (figure 3). Such a fixation means allows for maintaining the present device in a predetermined orientation. In particular, the functional orientation of the device is vertical or substantially vertical, with the upper element **2** placed upward, as it will be better described later on. The upper fixation means **21** can comprise to this end one or several holes such as threaded holes, so as to screw one or more brackets or flanges. Alternatively, the upper fixation means **21** can comprise clips adapted to cooperate with an structural support such as a bed armature. Other fixation alternatives can be envisaged, provided that the upper fixation means **22** maintains the device **1** at its functional position including during displacement of the patient's bed, where applicable, and physical contact with the surrounding environment. According to an embodiment, the upper fixation means **21** allows for an easy and removable fixation of the device on a support structure. To this end, it can comprise for example an manually articulated locking mean adapted to move from an open position, where the device is free, to a closed position, where the device is fixed on an appropriate support. Such a upper fixation means **21** may be arranged on a back face **23** of the upper element **2** for example. In the framework of the present description, the back face **23** of the device 1 is opposed to the front face **22,** which is accessible to the operators surrounding the patient. The back face **23** is thus oriented toward the support on which is fixed the device **1.** The lateral faces have their usual meanings, joining the back face and the front face.

The device according to the present invention comprises a reservoir **3** in which the fluid is collected. Such a reservoir **3** is arranged underneath the upper element **2.** The volume of the reservoir 3 is large enough to collect a body fluid for a period of time of at least half a day, preferably at least 24 hours, so that emptying operation of the reservoir is spaced enough. On the other hand, the reservoir remains compact so as to not hinder the space around the patient. The reservoir **3** can have for example the global shape of a parallelepiped rectangle, while other shapes can be envisaged. The volume can be between 200 ml and 2.5 litres, or between 300 ml and 1,5 litre depending on the needs. For the purpose of the present description, a reservoir may also designate a urinary bag, or a urinary recipient.

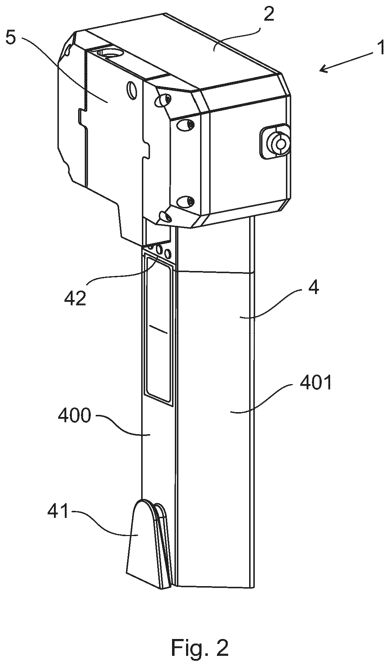

The device **1** of the present invention comprises an intermediate element **4** (better visible in figure 2), which is mechanically fixed to the upper element **2,** underneath thereof. The intermediate element **4** comprises one or several reservoir fixation means **41,** so that the reservoir **3** can be accommodated on the intermediate element. According to an embodiment, the reservoir fixation means **41** denote or comprise a hook on which the reservoir can be arranged. According to an embodiment, such a hook is placed at the bottom extremity of the intermediate element **4,** for example at a front face thereof. Alternatively or in addition, hooks may be arranged on lateral sides of the intermediate element **4** and/or at an upper position of the intermediate element. The reservoir **3** comprises at least one recess (not shown) adapted to cooperate with the hook or the hooks **41** of the intermediate element. Such a recess is arranged preferably on the back side of the reservoir **3.**

The reservoir **3** is preferably removably arranged on the intermediate element **4** so that it can be easily emptied and/or cleaned and replaced. In particular the reservoir can be handled with one hand, either for removing it from the device or for replacing it onto the device. To this end, the reservoir can comprise a handle **31** so as to be grasped and removed from the intermediate element **4** by a medical operator. Such a handle can take any suitable form, such as a rigid tab, a flexible link, or any equivalent. The handle may be fix or retractable. For ergonomic reasons, the handle **31** is preferably placed on the front side, or front upper portion of the reservoir **3.** It can however be arranged on a lateral side so as to save space and provide a more compact device **1.** Where necessary, the handle may be provided with a locking mechanism (not shown) such as a button adapted to activate a hook, comprising a spring. Such a hook may then be adapted to cooperate with a recess in the intermediate element so as to maintain the reservoir **3** onto the intermediate element **4.** Pushing the locking mechanism, for example with the thumb, while taking the handle may thus allow to disengage the hook from the intermediate element **4.**

Alternatively or in addition, the reservoir **3** can be provided with a valve **32** adapted to empty the reservoir **3** while it remains on the intermediate element **4.** Such a valve can be for example a rotative valve having two positions, including an open position to let the liquid flow out the reservoir and a closed position. According to an embodiment, the valve **32** may be connected to an output pipe **33** so as to direct the liquid in an external recipient or in a trash container. Alternatively, the output pipe **33** may be connected or connectable to an external device or recipient. The liquid can then be collected without removing the reservoir **3** from the intermediate element **4.** The valve **32** and the output pipe **33,** when present, may be arranged at a bottom position of the reservoir **3,** for example in the front side of the reservoir. For a better compacity, the valve may be arranged on a lateral portion of the reservoir **3,** either on a same side as a handle **31** or at the opposite side. In such a latter configuration, the handle arranged on one side can be used to incline the reservoir **3** toward the opposite side and allow for better emptying the reservoir through the output pipe **33.** According to another configuration, the valve **33** can be arranged underneath the reservoir **3.** The valve may be manually activable. Alternatively, the valve may be motorised or automatised so that the reservoir is automatically emptied, for example when a threshold level or threshold volume or a threshold time or a threshold weight is determined. According to an embodiment, an auto-siphon can be considered.

The reservoir **3** may be provided with a lid **34,** covering the top surface thereof, either completely or only partially. Such a lid **34** can thus be opened, while the reservoir **3** remains locked on the intermediate element **4.** Opening the lid **34** allows a visual check of the content of the reservoir **3.** In particular, the aspect or the colour of the liquid may be observed, at least to provide a qualitative information. The lid **34** can be linked to the side walls of the reservoir **3,** including the back side and the front side, by means of hinges. Alternatively, the lid **34** can be clipped onto the walls of the reservoir, either through lugs or by friction. The lid **34** may be provided with a handle adapted to open the reservoir.

The reservoir **3** can be provided with a vent **35** so as to avoid overpressure or under pressure within the reservoir **3,** in particular in case a lid is present. Such a pressure variation would otherwise impact the measure of the flow and/or the volume of liquid which is measured. The pressure variation here relates to a pressure difference between the pressure inside the reservoir and the external atmospheric pressure. The vent **35** can take the form of a through hole in the lid **34** or at an upper portion of the walls of the reservoir **3.** For an improve safety, and for avoiding contaminations, the vent can be comprise a membrane which avoids or limits leakage of fluid. While letting the pressure equilibrate, the membrane blocks the bacteria and other particles. Thus, only the air of the gas present in the reservoir **3** can escape or enter the reservoir **3.**

According to a preferred embodiment, the reservoir is free of lid and remains open. Such an arrangement can better suit, for example, some sanitary regulations.

According to another embodiment, the reservoir **3** is fully transparent.

The reservoir **3** is provided with an inlet **36** adapted to collect the fluid from the upper element **2** to the reservoir **3.** The reservoir inlet **36** is preferably arranged on the upper portion of the reservoir **3.** It can be for example provided on the lid **34.** This does not exclude that an inlet is provided on a lateral side or the front side or the back side of the reservoir. The fluid is collected from the upper element **2** by gravity. A reservoir inlet pipe **37** can be used to fluidly connect the upper element **2** with the reservoir **3.**

The reservoir inlet pipe **37** is preferably made of a flexible material such as a supple polymer. A silicone or equivalent polymer may be used. Such a flexible material limits or avoid the potential constraints between the reservoir **3** and the upper part **2** and thus does not impact the weighting of the reservoir **3.** According to an embodiment, the reservoir inlet pipe **37** is fixed to the reservoir inlet **36** at one of its extremities. It can be fixed to the upper element **2** at the opposite extremity. According to another embodiment, the inlet pipe **37** may be free in the reservoir inlet **36** so as to allow a free movement of the reservoir **3,** in particular for its weighting. Furthermore, a free inlet pipe **37** allows for easily removing the reservoir **3** from the intermediate element **4** and disconnecting the inlet pipe **37.** The inlet pipe **37** may then be connected again to the inlet **36** by sliding its extremity inside the inlet **36.**

The inlet pipe **37** may be oriented downward from the upper element **2** to the reservoir inlet **36,** with an angular inclination of between 5° and 80° with regard to an horizontal reference, or between 10° and 70°, typically between 20° and 60°. Thus, the collected liquid can naturally flow by gravity in the reservoir **3.** According to another embodiment, the inlet pipe **37** can be arranged vertically from the upper element **2** to the inlet **36** so as to avoid any stagnation of the collected liquid inside the pipe **37.**

According to an embodiment, the reservoir **3** can be made partly or completely with a rigid material such as a hard plastic or a hard polymer. Metallic material such as stainless steel or equivalent material may also be considered. According to an embodiment, only portions of the reservoir **3** are rigid and the remaining surface of the reservoir in flexible. Such non rigid, or flexible part can be made with a supple polymer. According to a preferred embodiment, the reservoir **3** comprises at least one rigid surface so as to establish a close contact with the intermediate element **4.** Typically, the back side **38** of the reservoir **3,** or part of its back side **38** which is in direct contact with the intermediate element is made of a rigid material.

According to an embodiment, the geometry of the reservoir **3** is complementary from the intermediate element **4.** For example, a longitudinal recess **39** can be provided on the back side **38** of the reservoir **3** so as to accommodate the intermediate element. In such a way, the device **1** presents a compact and ergonomic design. In addition, the lateral sides of the intermediate element **4** allows for better maintaining the reservoir 3 at its right orientation, avoiding any non desired inclinations to the right or the left side. The longitudinal recess **39** allows an ergonomic handling of the reservoir, in particular when positioning it and removing it from the intermediate element. The reservoir **3** can be easily placed against the intermediate element so as to cooperate with the fixation means **41.** Also, once it is locked out, it is easily removed from the intermediate element **4.**

The reservoir **3** may be completely opaque, for instance in case of metallic material or opaque polymer is used. Alternatively, the reservoir **3** may be at least partly transparent so that the collected liquid can be seen from outside without opening the reservoir. To this end a transparent window may be considered, either on the front side or on a lateral side of the reservoir. One or several linear transparent portions can be arranged for this purpose. Alternatively or in addition, the lid **34** can be transparent or partly transparent.

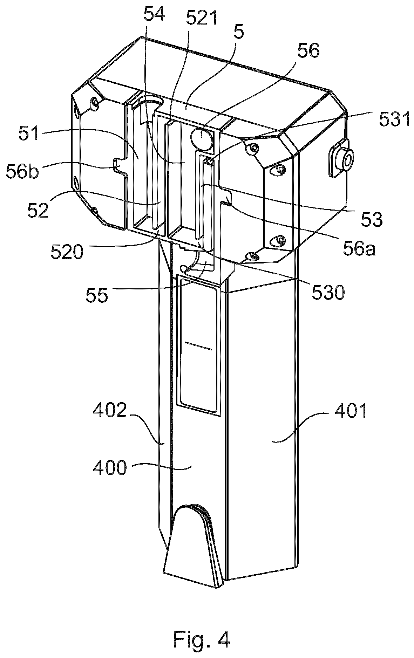

The device **1** according to the present invention comprises a flowmeter module **5,** better seen in figure 4. Such a flowmeter module **5** allows for measuring a flow of fluid, typically a urine flow. The flowmeter module 5 is preferably combined to the upper element **2** of the device **1.** Preferably, the flowmeter module **5** is removably accommodated onto or within the upper element **2.** To this end, it can be provided with one or several module fixation means **56a, 56b.** adapted to maintain the flowmeter module joint to the upper element **2.** Such module fixation means can comprise for example one or more lugs received in appropriate recesses of the upper element or the other way round, some recesses adapted to accommodate lugs of the upper element **2.** Other fixation means can be considered, provided that they allow an easy disconnection from of the flowmeter module **5** from the upper element **2.** According to a preferred embodiment, the flowmeter module **5** is placed on the front face **22** of the upper element **2.** It can be pressed against the upper element **2 to** be inserted into a dedicated housing. Alternatively, it can slide from the top or a lateral side of the upper element **2** until it reaches its position. An important aspect is that the flowmeter module **5** is fixed to the upper element 2 so as to resist any accidental external load or pressure, and potential pull from patient on the tube.

Alternatively, the flowmeter module **5** is integral with the upper element **2.**

The flowmeter module **5** typically comprises a central space where a volume of liquid can be temporarily stored up to a predetermined amount (Figure 4). Once an upper threshold of liquid is reached, the liquid can automatically flow out the flowmeter module **5** toward the reservoir, which is place underneath. The amount of liquid which is temporarily stored in the central space can be determined by a suitable sensor. According to a specific embodiment, the flowmeter module **5** comprises an inlet channel **51,** a first intermediate channel **52,** a second intermediate channel **53,** an outlet channel **55** and a central reservoir **54** arranged between the first **52** and the second **53** intermediate channels. The first **52** and the second **53** intermediate channels allow to maintain the liquid inside the central reservoir **54.** The first **52** and second **53** intermediate channels are typically defined by two vertical walls facing each other and each comprising an opening. For instance, the first intermediate channel **52** comprises a bottom first opening **520** and a top first opening **521.** The bottom first opening is oriented toward the inlet channel and the top first opening is oriented toward the central reservoir **54** so that the liquid collected in the flowmeter module through the inlet channel **51** passes through the first intermediate channel **52** up to the top first opening **521,** where it can be stored in the central reservoir **54.** The second intermediated channel **53** also comprises a bottom second opening **530** so that the liquid can flow from the central reservoir **54** to the second intermediate channel **53,** and a top second opening **531** on the opposite wall of the second intermediate channel **53.** The level of liquid stored in the central reservoir **54,** and also in the second intermediate channel **53,** increases up to the top second opening **531.** At this level, it is automatically siphoned out through an outlet **55,** arranged underneath the flowmeter module **5.** The liquid is guided to the reservoir **4** by mean of the inlet pipe **37,** joining the module outlet **55** and the reservoir inlet **36.** The central reservoir **54** is thus regularly empty automatically. The time of residence of the liquid in the central reservoir **54** thus depend on the flow of the liquid. The present of a liquid or the level of liquid in the central reservoir **54** can be automatically and periodically detected. Suitable sensors are implemented to this end. According to an embodiment, one or several capacitive sensors are arranged in close proximity the central reservoir **54** so as to determine the variation of the liquid level. Alternatively, an optical sensor is provided, either to sensed a level variation of the liquid within the central reservoir or an actual level of liquid. Such an optical sensor may be a laser optical sensor or a related device. The flowmeter module sensor or sensors, if any, can be arranged outside the flowmeter module **5.** They can be for example integrated in the upper element **2.** In case of an optical sensor, the flowmeter module **5** can be made of a transparent material or at least comprise a transparent window so that the level of liquid can be detected.

The time of residence of the liquid in the central reservoir **54** can be regulated. To this end, a porous material **56** can be arranged inside the central reservoir **54** so as to capture a certain amount of liquid and delay the syphoning operation. The porosity and the size of such a material can be determined according to the expected flow of liquid. Such a porous material can designate a membrane or a semi-porous element.

The device **1** according to the present invention further comprises a device inlet **57** allowing to collect the liquid from the external source, typically from the patient. The device inlet **57** can be fluidly connected to the patient by means of a standard urinary catheter (Not shown). According to a preferred arrangement, the device inlet **57** is placed on a top surface of the flowmeter module **5** so as to guide the liquid in the inlet channel **51** thereof. The flowmeter module **5,** the upper element **2** and the device inlet **57** form together a stable assembly. In particular, since the upper element **2** is fixed to an external support, the possible moves of the device inlet **57,** due to the patient moves, has no impact of the accuracy of the measurements.

The intermediate element **4** preferably takes the shape of a single housing linked to the upper element **2** (figure 2). Such a housing may have a square or a rectangular section. The shape of the housing may however be more diverse and present for example a round section, either circular or ovoid, or a trapezoidal section. The length of the intermediate element **4** is mainly determined by the height of the reservoir **3.** The length can typically be the same as the height of the reservoir or slightly higher. The bottom of the reservoir can coincide with the bottom of the intermediate element 4. Alternatively, the reservoir **3** can be fixed to the intermediate element 4 so that the bottom of the reservoir **3** is higher than the bottom of the intermediate element **4.** Under such condition, the device **1** can be placed on the floor without impacting the reservoir **3.** The width of the intermediate element **4** is typically lower that the width of the upper element **2** to which it is linked. The width of the intermediate element **4** is preferably also lower than the width of the reservoir **3.** The width of the intermediate element **4** may correspond to one tenth to more than four fifth of the width of the reservoir **3.** A larger width increases the surface contact with regard to the reservoir **3.** Furthermore, a larger width improves the robustness of the device **1** and provides a better stability of the reservoir **3** once it is arranged on the intermediate element **4.** The intermediate element **4** thus takes a central position with regard to the back side **38** of the reservoir **3.**

According to an embodiment, the intermediate element **4** comprises two or more than two longitudinal housings instead of only one longitudinal housing. In this case, the two or more longitudinal housings may be join at their upper extremity so as to be linked to the upper element **2.** Alternatively, the two or more housings are separately connected to the upper element **2.** The two distinct parts of the intermediate element **4,** where applicable, can be both arranged on the back side of the reservoir **3.** Alternatively, they can be lodged each on a lateral side of the reservoir **3,** with the advantage that the reservoir can be maintained between the two opposite parts of the intermediate element **4.** The intermediate element **4** may alternatively take the form of a large plate covering most of the back side **38** of the reservoir **3.** Independently of the shape of the intermediate element **4,** the reservoir **3** is removably arranged on it.

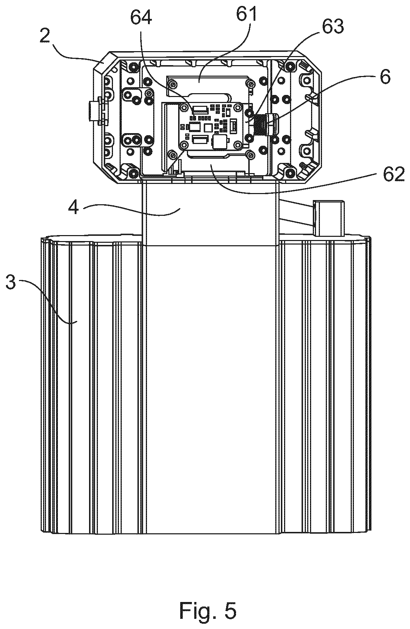

The intermediate element **4** is preferably linked to the upper element **2** by means of a weight sensing device. Such a weight sensing device can comprise a strain gauge load cell or an other equivalent sensor usable as a weight sensor. The weight sensing device can be for example a dynamometer **6** (figure 5) having a bottom arm **62** mechanically linked to the intermediate element **4** and an upper arm **61** mechanically linked to the upper element **2.** The bottom arm **62** and the upper arm **61** can be join together by a central arm **63.** The dynamometer **6** is arranged inside the upper element **2** so that the bottom arm **62** and the upper arm **61** are arranged in a vertical plan when the device **1** is in use. The traction force applied to the weight sensing device allows for determining the weight of liquid inside the reservoir **3.**

According to a preferred embodiment, the dynamometer above-described is designed for a traction of more than 50 kg, or more than 80 kg or more than 100 kg or even more than 150 kg. The characteristics of the dynamometer **6** are thus significantly over-dimensioned for the usage of the present device **1.** This represents an advantage of providing a higher robustness, in particular in the hospital environment where medical operators may hit the device. The counterpart may be a lack of accuracy in the weight measuring. In addition, the dynamometer may be more sensitive to temperature variations. So as to compensate these drawbacks, the weight sensing device can comprise or be connected to a correcting unit **64** adapted to adjust the weight sensed by the dynamometer to a real weight. Such a correcting unit **64** typically comprises computing means and algorithms suitable for this purpose. The weight sensing device can in addition comprise or be connected to a thermometer **6'** adapted to sense the temperature of the dynamometer **6.** Such a thermometer can be on any type, including contactless temperature sensor, ambient air thermometer localized by the dynamometer. Preferably, the sensed temperature is transmitted to the correcting unit **64** so that the sensed weight is properly adjusted. Other correcting sensors such as a humidity sensor may also be considered.

According to another embodiment, the weight sensing device comprises more than one dynanomter **6** as above-described. In case several distinct housing form the intermediate element **4,** each one being individually linked to the upper element **2,** then a distinct dynamometer is provided for each of the housings. The weight can be determined individually. The combined measurements can be further corrected or compute by the correcting unit. For example an average weight may be computed between the weight of both intermediate elements **4.**

According to another embodiment, several dynamometers **6** as above-described are arranged in parallel, even for a single housing having a single link to the upper element **2.** The measurement of each of the dynamometer can then be computed and eventually corrected by the correcting unit **64.** Such a solution allows for using more sensitive dynamometers while keeping a global robustness.

Although a dynamometer is here described as a weight sensing device, this does not exclude to use other type of weight sensor. For example piezo elements may be considered instead or in combination.

According to an embodiment, weight variation is measured instead of an absolute weight.

The device according to the present invention further comprises a means to determine the presence and/or the level of liquid in the reservoir **3.** Such a liquid sensing device may comprise or denote an optical device directed to the surface of the liquid in the reservoir **3.** For example, such an optical sensing device can be lodged underneath the upper element **2** and oriented downwards to focus on the content of the reservoir. The reservoir can be to this end transparent to such optical means. Alternatively or in addition, the liquid sensing device can be of the type of capacitive devices, comprising at least one electrode.

According to an embodiment, several electrodes are arranged horizontally in the intermediate element **4** close to the surface of the reservoir **3,** preferably close to a rigid surface of the reservoir **3.** Such electrodes can form a capacitive sensor. Horizontal electrodes can be gradually arranged along the intermediate element **4** so that the number of activated electrodes increases with the volume of liquid collected in the reservoir **3.**

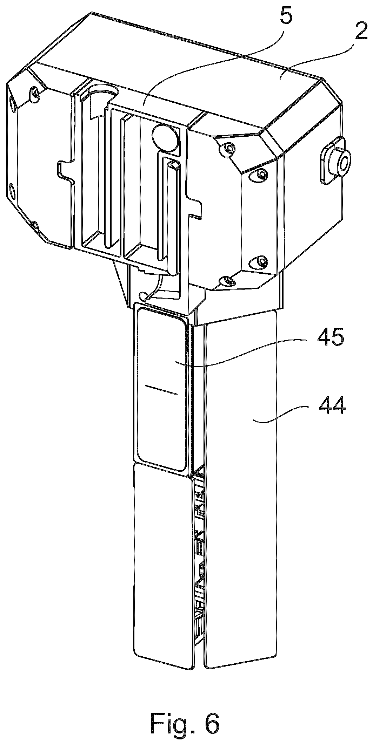

According to an alternative arrangement, better visible in figure **6****,** one or several electrodes **44** are oriented vertically close to a rigid surface of the reservoir **3,** typically close to the back side of the reservoir **3.** Such vertical electrode or electrodes can be place on a surface or behind a surface of the casing of the intermediate element **4.** For example, if the casing has a sectional trapezoidal geometry, with the largest side oriented outward, the electrodes may be arranged on the lateral surfaces, oriented toward the reservoir **3.** Several electrodes **44** can be arranged at the same height and cover a same length of the intermediate element **4.** In such an arrangement, all the vertical electrodes may cover substantially the full length of the intermediate element **4** or at least half or the two third or the three quarters of it.

Alternatively, several vertical electrodes **44** can be offset from one another. In such an arrangement, electrodes are shifted upward. They may individually cover a limited length of the intermediate element **4,** such as a 10^{th} or a 5^{th} of the length of the intermediate element **4.** However, several electrodes together can cover the full length of the intermediate element **4** or substantially the full length or at least half of it, or at least half or the two third or the three quarters of it.

Graduated activation of the electrodes **44** may indicate the level of the liquid collected in the reservoir **3,** whether they are arranged horizontally or vertically.

According to an embodiment, two or more vertical electrodes having the same length are arranged at the same height of the intermediate element **4.** For example, one vertical electrode **44** can be placed on each of the side surfaces of the intermediate element **4.** Under such a configuration, an electrode command unit **440** is adapted to emit a signal to the electrodes **44** at a given input frequency and collect a signal of an output frequency from the electrodes. The collected output frequency allows to determine the level and/or the change of level of the liquid in the reservoir **3.**

Such a frequency analysis can be combined with a graduated activation of the electrodes due to their shift so as to improve the sensed level of liquid.

According to an embodiment, the present device **1** comprises additional sensors **8** adapted to determine its position, its move and/or its orientation. One or mode accelerometers may be arranged either in the upper element **2** or in the intermediate element **4** or in both the upper element **2** and the intermediate element **4.** Such accelerometers may be three-dimensional accelerometers. This however does not exclude the use of accelerometers of one or two dimensions. Such combined accelerometer can help detecting an adverse orientation of the device **1,** typically an inclined orientation, which deviates from the functional vertical orientation.

One or more of the accelerometers **8** further help determining vibrations or move of the device **1,** for example when the bed of a patient is moved from one room to another one.

Alternatively or in addition, the reservoir **3** may also be provided with such a sensor adapted to determine its position and/or orientation. The relative orientation of the reservoir **3** and the upper element **2** may thus be determined. In case the reservoir **3** is not well engaged in the dedicated fixation means **41** or is offset its functional position, then a alert can be provided.

Signals provided by the accelerometer(s) or other equivalent sensors adapted to determine the position, movement and orientation of the device or part of the device, are collected by a central unit **7.**

It is thus highlighted that the present collecting device comprises a combination of different sensors, in particular different sensors of different types. It comprises a combination of at least two different sensors, or even three or more than three different sensors. The sensors are selected among a flowmeter module **5,** one or several capacitive sensors, optical sensors, weight sensor such as a dynamometer **6,** a temperature sensor, humidity sensor, accelerometer. Such a combination of sensors allow to confirm the measurements of each of those sensors and detect errors in case of discrepancies. The combination of sensors also allows to define the right time frame in which measurement are triggered or to define whether the data are reliable and can be collected and further computed.

The device **1** according to the present invention may comprise additional elements such as a battery **45,** preferably a rechargeable battery. The battery can be lodged in the housing of the intermediate element **4.** The device is provided with an electrical connexion at least for recharging the battery **45.** The device **1** can comprise an alarm system, in particular a visual alarm system such a LED or a LED assembly **42.** Such a visual alarm can replace the usual audio signals and allow for a more efficient localisation of the default. This is particularly advantageous in case several medical devices or localised in a room and susceptible to emit alert sounds. The LED or the LED assembly can comprise light emitting systems of the type of ultra bright LED. Various colors of LED can be arranged so as to visually indicate the nature of the default or its gravity. For example, Visual alerts can be provided in case of accidental move of the reservoir or deviation of the device **1** or over pressure on the reservoir due to external objects. A specific visual alert can be provided when the reservoir needs to be empty, or when the battery needs to be recharged.

According to an embodiment, the device is provided with at least one data transmission means **47,** adapted to transmit the sensed data to an external unit **91.** The data can be related to the sensed flow of liquid, to the amount or volume of liquid, to the variation of the flow of liquid collected in the reservoir **3.** Data also comprise error or fault data related to discrepancies between several sensors of the device **1.** For example, the data can comprise a confidence evaluation of the measured liquid flow or liquid amount. For example, discrepancies between the weight measurement of the liquid by the weigh sensing device and by the liquid sensing device can be recorded and transmitted. Data transmission means **47** can comprise usual wireless protocols such as WiFi or Bluetooth. Alternatively, transmission means such as ISM can be used to avoid interferences with other medical devices in the room. The ISM frequencies can for example of 800 MHz or 900 MHz or any other suitable frequences.

The sensed data can be directly send to an external computing unit, so as to be computed and displayed on a dedicated screen. According to a preferred embodiment, the present device **1** is exempt from display and/or electronic command buttons. The device **1** thus remains compact and easy to use. According to an embodiment, the sensed data can be partially or completely processed before sending data to an external computing unit. Typically, a first process level can be performed within the device **1,** such as a control of the reliability of the sensed data or the material performances. Based on this control, the processed data can be sent to the external computing unit so as to be displayed or further processed. According to an embodiment the internal process is performed by a central unit **7,** arranged inside the device **1.**

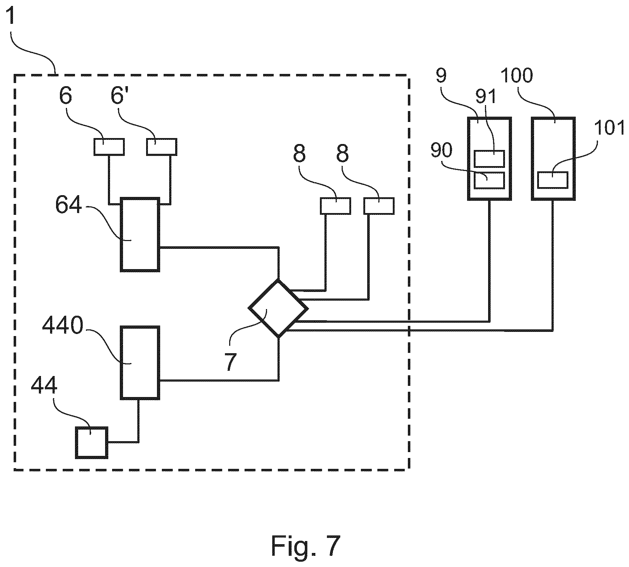

The central unit **7** can be directly connected to the sensors present in the device **1,** including the accelerometers **8,** the weight sensing device **6, 6',** the capacitive sensors (Figure 7). According to such an arrangement, the electrode command unit **440** and the correcting unit **64** may be integrated to the central unit **7.** Alternatively, the electrode command unit **440** and the correcting unit **64** are separate units connected to the relevant sensors and further connected to the central unit **7.** This does not exclude that individual sensors are in addition connected to the central unit **7.**

The present invention also relates to a system comprising one or several devices **1** as above-described. The system comprises in addition at least one remote display **90** adapted to display the sensed data. Such a remote display **90** can be integrated to a monitoring device **9** already present in the medical room. The data displayed on the remote display **9** can then be used to monitor the liquid flow and/or the liquid amount sensed by the device **1.** In a preferred embodiment, the display is not embedded to the device here-described so as to simplify its handling and for improving its compactness. Other data such as battery charge or the necessity of emptying the reservoir **3** can also be displayed. Any other relevant data can be displayed. Alternatively or in addition, a remote display denotes a screen that a medical agent can monitor outside the patient room, for example in a control medical room adapted to monitor several patients or several rooms. According to an embodiment, the device **1** can be paired with a monitoring device **90** so as to be recognized and coupled to it. Thus, data can be exchanged between the device **1** and the remote display **90.** According to an embodiment, the monitoring device **9** also comprises a external unit **91** adapted to receive and compute the data send by the device **1.** Alternatively, the external unit **91** denotes a computer or a computing device distinct from any monitoring device.

Several devices **1** can be connected or paired with a given display **90** and/or a given external unit **91.**

The system of the present description can further comprise one or several injection and/or perfusion devices **100.** Such injection and/or perfusion devices are fluidly connected to the patient so as to inject a certain amount of a liquid, either sequentially or in a continuous way. The flow and/or the volume of liquid injected to the patient can be monitored by a dedicated input unit **101.** The input unit **101** can then transmit the corresponding data to the central unit **7.** Alternatively, the central unit **7,** is connected to such injection and/or perfusion devices so as to determine the amount and/or the flow of liquid injected to the patient. Alternatively, the amount and/or flow of liquid injected to the patient is transmitted to the external unit **91** so as to be computed externally to the device 1. Such an arrangement provides the advantage of limiting the electrical consumption of the device **1.**

According to an embodiment, the present system allows to compare the amount and/or flow of liquid injected to the patient and the amount and/or flow of liquid collected by the device **1.** In case a significant difference is detected, an alarm signal can be provided. Such a difference may denotes significantly less fluid collected in the reservoir than injected in the patient's body, which can indicate a water retention. Alternatively, such a different may indicate a significant increase of fluid collected in the reservoir **3** compared to the liquid injected in the patient's body. The difference, either positive or negative, can be considered significant above a threshold of around 10% or around 20% or around 30%, between the collected fluid and the fluid injected to the patient.

It is highlighted that either the device **1** or the system comprises means to receive and compute data sensed by the device **1** and data related to the fluids injected in the patient body and to compute some differences between both datasets. Such a means can be generically designated as a computing module adapted to compute the overall data and identify significant differences between fluids injected in the patient and fluid collected by the device **1.** Such a computing module can be either the external unit **91,** the central unit **7,** the input unit **101,** or a combination thereof either complete or partial. One skilled in the art understands that the above-mentioned external unit **91,** central unit **7** and input unit **101** can be physically distinct or integrated into a single element. These external unit **91,** central unit **7** and input unit **101** are provided with the necessary computing means including software, memory and data transmission means.

According to an embodiment, such a computing module comprises or is connected to an artificial intelligence module, a deep learning module or a combination thereof, or any equivalent means.

The present invention further relates to a method or a process of sensing a flow and/or volume of a fluid output from a patient. In particular the urine outflow. The method comprises a step **E1** of measuring a flow of fluid outputted from a patient. The flow measurement is determined for instance by the flowmeter module **5** above described. The method comprises a step **E2** of determining the weight of liquid collected into the reservoir 3. The weigh of liquid can be determined for example by means of a weigh sensing device as above described.

The data collected during the steps **E1** and **E2** can be sent to a computing unit such as the external unit **91** or the central unit **7.** Each data set can be sent on a continuous basis or at regular intervals to such a computing unit. The method further comprises a step **E3** of comparing data resulting from step **E1** and step **E2** and identify potential discrepancies. Discrepancies indicate that the measure flow and the measure volume are not consistent. Such discrepancy may be due to a load on the reservoir or to a misposition or a misorientation of the device or the reservoir **3.**

The method further comprises a step **E4** of determining the position and /or the orientation of the device **1** or parts of the device **1,** in particular the reservoir **3** and the upper element **2.** Such position and/or orientation is determined for example by means of the accelerometers present on the device **1.** The collected data are sent to a computing unit such as the external unit **91** or the central unit **7.**

According to an embodiment, the computing unit uses the position and/or orientation of the device to confirm a discrepancy between the flow and the volume determined during the steps **E1** and **E2.** In such extend, the measurement of the position and/or orientation is triggered only in case a discrepancy is identified. Alternatively, the measurement of the position and/or orientation is continuously measured or measured at regular intervals and sent to the relevant computing unit. In such extend, the position and/or orientation of the device is combined to the data related to the sensed flow and/or volume of the outputted fluid so as to validate their accuracy in case the position and the orientation of the device is acceptable, or invalidate their accuracy in case position or the orientation is considered wrong.

The method further comprises a step **E5** of sensing moves or vibration of the device **1.** Such a move or vibration can be sensed for example by the one or more accelerometers present in the device **1.** The corresponding data can be sent to the computing unit, whether it is the external unit **91** or the central unit **7.** According to an embodiment, the corresponding data is transmitted only in case a move or vibration are detected. Alternatively, a continuous data is transmitted even in absence of move or vibration, so that the accuracy of data measured in step **E1** and/or **E2** can be confirmed in absence of move or vibration or invalidated in case a move or a vibration is sensed. According to a further embodiment, the steps **E1** and **E2** of measuring the flow and/or volume of fluid is triggered only when no move neither vibration is sensed. Alternatively, data are continually sensed in step **E1** and **E2** and transmitted only in case no move neither vibration is detected.

In case discrepancies between the measurement of flow and volume of outputted liquid, or in case of invalid measurement, for example due to wrong position or orientation or moves or vibrations, a step **E6** of emitting a first alert can be triggered. Such a first alert can be a sound or a light emission, for example by means of the LED assembly **42** above-described or a combination thereof. The step **E6** can also be triggered in case other parameters are sensed, such as a low charge of the battery indicating that it needs to be recharge, or a high level of liquid in the reservoir **3,** indicating that the reservoir needs to be empty.

When the sensed data related to the flow and/or volume of the outputted fluid is considered valid, then it is implemented as such in the relevant computing unit, being either the external unit **91** or the central unit **7.**

The method further comprises a step **E7** of determining the flow and/or amount of liquid injected in the patient by means of the above-mentioned injection devices. The corresponding data is transmitted by the input unit **101** to the relevant computing unit, being either the external unit **91** or the central unit **7.** In addition, step **E7** may comprise the name, the nature and/or the concentration of the products injected in the patient's body.

According to an embodiment, when a significant difference between the detected outputted flow and/or volume of fluid and the liquid flow and/or volume injected to the patient's body is detected by the relevant computing unit, being either the external unit **91** or the central unit **7,** a second alert can be triggered. Said second alert is preferably different from the first alert above-mentioned. In particular, the second alert highlight a potential medical issue of the monitored patient. The second alert can be a sound and/or an optical signal, for example emitted by means of the LED assembly **42** above-described or a combination thereof. In addition, messages can be sent to dedicated persons such as medical doctors, nurse or other care givers.

According to an embodiment, events related to either the first or the second alerts can be stored in the relevant computing unit, being either the external unit **91** or the central unit **7,** for further access and analysis.

According to an embodiment, the present method further comprises a step **E8** of soring data and identifying specific patterns. The patterns may identify a combination of the type and/or the concentration of the products injected into the patient's body, the differences between the injected flow and/or volume. The pattern may comprise other parameters according to the needs. The pattern may be identify by an artificial intelligence module.

According to an embodiment, different sizes of reservoir **3** can be arranged on the intermediate element **4.**

According to an embodiment, the present device comprises an horizontality indicator such as a visual indicator, allowing to determine the inclination of the device with regard to the verticality. Alternatively or in addition, the upper fixation means **21** comprise hinges so that the device take a vertical position independently on the inclination of the external support on which it is fixed.

According to an embodiment, the lid **34** of the reservoir **3** denotes a bevel surface instead of a plane horizontal surface. Such inclined surface limits the risks of an accidental overload of the device by putting weigh on the lid. Such heaving objects would slide out of the lid instead on remaining on it. Alternatively or in addition, the device can comprise a protecting surface above the reservoir **3,** said protective surface being linked to the fix upper element **2.** By this way, any accidental weight would not impact the device, and in particular the weight sensing device.

and a flowmeter module **5,** wherein said intermediate element **4** is connected to said upper element **2** underneath thereof, and said reservoir is removably combined to said intermediate element **4,** wherein said flowmeter module **5** is removably combined to said upper element **2** and fluidly connected to said reservoir **3** said device comprising at least two sensors among one or several weight sensor, one or several accelerometer, one or several optical sensors, and one or several capacitive sensors.

### reference symbols in the figures

- 1: Fluid meter device
- 2: Upper element
- 21: Upper fixation means
- 22: Front face
- 23: Back face
- 31: Handle
- 32: Valve
- 33: Output pipe
- 34: Lid
- 35: Vent
- 36: Reservoir inlet
- 37: Inlet pipe
- 38: Back side of the reservoir
- 4: Intermediate element
- 41: Fixation means
- 42: LED assembly
- 44: Electrode
- 45: Battery
- 47: Data transmission means
- 440: Electrode command unit
- 5: Flowmeter module
- 51: Inlet channel
- 52: First intermediate channel
- 520: Bottom first opening
- 521: Top first opening
- 53: Second intermediate channel
- 530: Bottom second opening
- 531: Top second opening
- 54: Central reservoir
- 55: Module outlet
- 56: Porous material
- 56a, 56b: Module fixation means
- 57: Device inlet
- 6: Dynamometer
- 6': Thermometer
- 61: Upper arm
- 62: Bottom arm
- 63: Central arm
- 64: Correcting unit
- 7: Central unit
- 8: Accelerometer
- 9: Monitoring device
- 90: Remote display
- 91: External unit
- 100: Injection devices
- 101: Input unit

## Claims

1. A system adapted for monitoring an output flow and/or amount of liquid from one or more patients with regard to an input flow and/or amount of liquid injected to said one or more patient's body, comprising:
- at least one collecting device (1) comprising a combination of sensors adapted for measuring a flow and/or a volume of a liquid collected from said one or more patients,
- a unit adapted to receive and compute data sensed by said combination of sensors of the at least one collecting device (1),
- one or several perfusion and/or injection devices fluidly connected to the one or more patients so as to input a determined flow and/or amount of a liquid,
- one or more input units (101) connected to said one or several perfusion and/or injection devices, and adapted to transmit flow and/or volume data of the liquid injected to said one or more patients, and
a means to receive and compute data sensed by the collecting device (1) and data related to the fluids injected in the patient body and to compute some differences between both datasets.

2. System according to claim 1, further comprising a display external and remote to the collecting device (1).

3. System according to one of claims 1 and 2, wherein said combination of sensors denotes a combination of at least two sensors selected among a flowmeter module (5), one or several capacitive sensors, optical sensors, weight sensor such as a dynamometer (6), a temperature sensor, humidity sensor, and accelerometer.

4. System according to one of claims 1 to 3, wherein the collecting device (1) comprises an upper element (2), a reservoir (3), an intermediate element (4), and a flowmeter module (5), wherein said intermediate element (4) is connected to said upper element (2) underneath thereof, and said reservoir is removably combined to said intermediate element (4), wherein said flowmeter module (5) is removably combined to said upper element (2) and fluidly connected to said reservoir (3).

5. A collecting device adapted to collect an output fluid from a patient and measure the flow and/or volume of said collected fluid, said collecting device comprising an upper element (2), a reservoir (3), an intermediate element (4), and a flowmeter module (5), wherein said intermediate element (4) is connected to said upper element (2) underneath thereof, and said reservoir is removably combined to said intermediate element (4), wherein said flowmeter module (5) is removably combined to said upper element (2) and fluidly connected to said reservoir (3), and wherein said collecting device (1) comprises a plurality of sensors selected among a flowmeter module (5), one or several capacitive sensors, optical sensors, weight sensor such as a dynamometer (6), a temperature sensor, humidity sensor, and accelerometer.

6. Collecting device according to claim 5, wherein said one or several capacitive sensors comprises at least one electrode (44) arranged on said intermediate element in close proximity of the reservoir (3) and an electrode control unit adapted to supply a signal to said electrode at a determined frequency and to collect the response signal receive by said electrode.

7. Device according to one of claims 1 or 2, said one or several weight sensor comprises a dynamometer arranged in the upper element (2) and connected to said intermediate element (4), said dynamometer comprising a strain gauge load cell, said weight sensor further comprising a load control unit and a thermometer, wherein said load control unit is adapted to correct the weight sensed by the dynamometer according to the sensed temperature value.

8. Device according to one of claims 1 to 3, wherein said flowmeter module (5) comprises an inlet channel (51), a first intermediate channel (52), a second intermediate channel (53), an outlet channel (55), a central reservoir (54) arranged between the first (52) and the second (53) intermediate channels and a flow regulation membrane (56) arranged inside said central reservoir (54).

9. Device according to one of claims 1 to 4, wherein said flowmeter module (5) further comprises fixation means (56a, 56b) adapted to removably combine said flowmeter to said upper element (2).

10. A method of measuring a flow and/or volume of a fluid output from a patient by means of a system according to one of claims 1 to 5, said method comprises:
- A step E1 of measuring a flow of fluid outputted from a patient, by means of a fluid meter module (5),
- A step E2 of determining the weight of liquid collected, by measuring the weight of the reservoir (3) of the device (1) by means of a weigh sensing device,
- A step of sending the data obtained in steps E1 and E2 to a relevant computing unit, and a step E3 of comparing the data resulting from step E1 and step E2 so as to identify potential discrepancies.

11. A method according to claim 10, further comprising a step E4 of determining the position and /or the orientation of the device (1) or parts of the device (1), so as to confirm a discrepancy or validate the data obtained in steps E1 and E2.

12. The method according to one of claims 10 and 11 further comprising a step E5 of sensing moves or vibration of the device (1), so as to confirm the data obtained in steps E1 and E2 in case of absence of moves or vibration, or triggered step E1 and E2 only in case of absence of moves or vibration.

13. Method according to one of claims 10 to 12, further comprising a step E6 of emitting a first alert in case of discrepancies or in case of a wrong position and or orientation of the device 1.

14. Method according to one of claims 10 to 13, further comprising a step **E7** of determining the flow and/or amount of liquid injected in the patient by means one or more injection devices, and comparing the same with the data related to the output flow and/or fluid of the patient so as to identify possible differences and or pattern.
